# EUROPEAN PATENT SPECIFICATION

(11) **EP 0 842 914 B1**
(45) Date of publication and mention of the grant of the patent: **11.12.2002**
(21) Application number: 97120167.8
(22) Date of filing: 18.11.1997
(51) Int. Cl.: C07C 2/66

(54) **Alkylation process with enhanced catalyst stability**
Alkylierungsverfahren mit höherer Katalysatorstabilität
Procédé d'alkylation ayant une plus grande stabilité du catalyseur

(30) Priority: 18.11.1996 US 749524
(43) Date of publication of application: 20.05.1998
(73) Proprietor: FINA TECHNOLOGY, INC., Houston, Texas 77267-4412 (US)
(72) Inventor: Kuchenmeister, Mark E., Friendswoord, Texas 77546 (US); Butler, James R., Houston, Texas 77059 (US)
(74) Representative: Leyder, Francis

(56) References cited:
- EP-A- 0 148 147

## Description

This invention relates to a process for the alkylation of aromatic compounds using a silicalite catalyst wherein the stability of the catalyst is enhanced by the addition of water to the reaction zone.

Alkylaromatic compounds, such as ethylbenzene, ethyl toluene, isopropylbenzene and the like are very important as precursors in the manufacture of vinylaromatic monomers. The resulting vinylaromatic monomers are used to make a variety of useful polymer materials, styrenic resins for example. In a typical commercial process, alkylaromatic compounds are produced by catalytic alkylation at elevated temperatures.

Developments in the catalysts employed in the above-referenced alkylation process have improved the efficiency of the commercial manufacture of alkylaromatic compounds. One group of particularly effective catalysts is that of the pentasil materials. Pentasils, including the ZSM-5 and silicalite type zeolites, are examined in detail by Debras et al. in "Physico-chemical characterization of pentasil type material, I. Precursors and calcined zeolite, and II. Thermal analysis of the precursors", *Zeolites,* 1985, Vol. 5, pp 369-383. Both silicalite and ZSM-5 type catalysts can be characterized very generally in terms of mole oxide ratios as follows:

x M₂O:Al₂O₃:y SiO₂

Wherein:
- M: is an alkali metal cation, normally sodium
- x: is the mole ratio of alkali metal oxide to alumina, and
- y: is the silica/alumina ratio.

Although both silicalite and ZSM-5 zeolites are categorized as pentasils, the ZSM-5 materials are characterized as aluminosilicates as disclosed in U.S. Patent No. 3,702,886 to Argauer et al. or as metal organosilicates as disclosed in Re 29,948 to Dwyer (high silica/alumina ratio). Silicalites, on the other hand, are described in U.S. Patent No. 4,061,724 to Grose et al. The silicalite type pentasil contains minor amounts of aluminum as an impurity only. The aluminum content of a silicalite is less than one aluminum atom for each unit cell of 96 SiO₂ tetrahedra. Thus, silicalite has a silica/alumina ratio of about 200 or more. Debras et al., *supra*, details several differences between the two materials, including differences in structure and synthesis procedure.

As disclosed in EP-A-148,147, silicalite of monoclinic symmetry can be used in the alkylation of an aromatic substrate such as ethylbenzene or toluene. The monoclinic silicalite can be produced by calcining silicalite of orthorhombic symmetry in order to convert the crystal symmetry to monoclinic. The silicalite crystals so obtained have crystal sizes of up to 10 microns with crystal sizes between 1 and 3 microns and between 4 and 6 microns being specifically disclosed. The silicalite crystallites may be mixed with a suitable binding material such as aluminum hydroxide to make catalyst pellets.

One problem encountered in alkylation processes using a catalyst is that the catalyst loses its ability to promote alkylation over time. That is, over time the catalyst alkylates progressively less and less of the aromatic feedstock. The alkylation unit must be finally shut down and the catalyst regenerated or replaced, at substantial expense to the chemical manufacturer. This loss of catalytic activity is thought to be due to the build-up of long chain polymers on the surface and in the pore structure of the catalysts. This build-up of polymers is commonly referred to as "coking".

Although the pentasil type catalysts have improved alkylation/transalkylation characteristics, coking remains a problem in alkylation processes employing them. In particular, it has been widely noted that even small amounts of water produce the degeneration of the ZSM-5 type catalysts. (See, for example U.S. Patent No. 4,387,260 to Watson et al., U.S. Patent No. 4,490,570 to Forward et al., and U.S. Patent No. 4,559,314 to Shihabi). It is generally believed that water progressively dealuminates the framework of the zeolite crystals, irreversibly deactivating it. Common industrial procedure calls for aromatic feedstock to be subjected to a dehydrating procedure prior to initiation of the alkylation process when ZSM-5 catalyst is used.

On the other hand, U.S. Patent No. 4,387,260 to Watson et al. discloses the alkylation of aromatic compounds over silicalite catalyst wherein a water cofeed is supplied, preferably with in the range of 20,000-60,000 ppm water. U.S. Patent Nos. 4,774,379 to Butler et al. and 4,387,260 to Watson et al. also disclose alkylation processes in which water is added to extend the effective life of a silicalite catalyst. It should be noted, however, that in the case of the steam co-feed disclosed in U.S. Patent No. 4,387,260 the catalyst ages are reported in hours, and some catalyst degradation was reported as the steam was co-fed.

Thus, improved alkylation processes are needed that give enhanced stability of silicalite catalysts over time.

In accordance with the present invention, there is provided a process for the alkylation of aromatic substrates by alkylating agents using silicalite catalysts in which water is co-fed to the reaction zone at concentrations from 100 - 60,000 ppm to enhance catalyst stability. Tests with a silicalite catalyst having a crystal size within a specified range showed catalyst stability levels superior to those reported in the previous references *supra.* As a result of the process of the present invention, it is no longer necessary to dry the aromatic feed prior to alkylation over silicalite catalysts; a discovery resulting in significant cost savings in plant operation.

To carry out the process of the present invention, the aromatic feedstock and water are supplied to a reaction zone and brought into contact with a silicalite type catalyst. An alkylating agent is also supplied to the reaction zone which is preferably operated under temperature and pressure conditions to maintain the aromatic substrate in the vapor phase.

The operating conditions are selected to promote the alkylation of the aromatic substrate in the presence of the silicalite catalyst, including a temperature of from about 250°C to about 500°C and 13,79 to 34,47 bars (200 psig to 500 psig) pressure.

A preferred application of the present invention is the alkylation of benzene with ethylene to produce ethylbenzene. The process is preferably carried out in the vapor phase. The process of the present invention may be carried out in a multiple bed reactor wherein there is a stoichiometric excess of benzene supplied to the reactor relative to the ethylene concentration. In the preferred embodiment, the reactor effluent is subjected to successive fractionations to separate benzene, ethylbenzene, polyalkylated compounds and heavy residues into separated streams. The ethylbenzene product may be recovered after separation.

The stream of polyalkylated compounds is directed to a separate transalkylation reactor or is recycled to the alkylation reactor for transalkylation into the desired monoalkylated product simultaneous with the alkylation of the aromatic feedstock.

In one embodiment of the present invention, the catalyst comprises a silicalite characterized as 70-100% monoclinic and 0-30% orthorhombic silicalite formulated with 10 - 0% refractory oxide binder. Alumina has been found to be a suitable binder. The silica/alumina ratio is 50-500 and the average crystal size less than 0.50 µm. The catalyst is further characterized by a maximum pore size of the refractory oxide binder of 1000-1800 Angstroms and a sodium content of the silicalite of less than about 50 ppm.
FIGURE 1 is a schematic representation of the process of the present invention;
FIGURES 2 - 4 are graphical representations showing experimental results of co-feeding 3% steam to a silicalite catalyst with reduced particle size.

The process of the present invention can be carried out using a variety of process equipment, including a reactor vessel which defines a reaction zone containing catalyst material. Either single or multiple catalyst beds may be employed in the reaction zone. In the case wherein benzene is alkylated with ethylene to produce ethylbenzene, the ethylene and benzene reactants may be mixed and preheated prior to introduction to the reaction zone. The reaction zone may consist of one or more catalyst beds where the reactants contact the catalyst under reaction conditions. The reaction products are withdrawn from the reaction zone after a controlled residence time and then collected and separated by conventional techniques.

Products of the alkylation reaction include ethylbenzene, unreacted benzene, polyalkylated aromatics and heavy residue. Excess benzene along with polyalkylaromatics are typically recycled to the reaction zone and the heavy residue withdrawn as a purge stream. In the alternative, the polyalkylaromatics may be directed to a separate transalkylation reactor where they are broken down into ethylbenzene.

The catalysts used in the process of the present invention are selective to the production of ethylbenzene in a temperature range of from about 250°C to about 500°C and a pressure range of from about 13.79 bars (200 psi) to about 34.47 bars (500 psi). The alkylation reaction is exothermic, resulting in a positive temperature differential from the reactor inlet to the reactor outlet of about 20°-100°C.

The mole ratio of alkylating agent to the alkylatable aromatic substrate may be varied in accordance with the desired reaction product. Normally, a substantial stoichiometric excess of aromatic feedstock relative to the alkylating agent will be employed. For example, when ethylene is employed in the alkylation of benzene to produce ethylbenzene, the mole ratio of the alkylatable substrate to ethylene should be within the range of 2:1-20:1 benzene to ethylene. In any case, there should be a substantial stoichiometric excess of the alkylatable substrate.

Another critical factor in alkylation/transalkylation reactions is the time that the reactants are in contact with the catalyst bed, expressed as space velocity. The space velocity based upon the alkylatable substrate will normally be within the range of 30-200 hours⁻¹. Further description of the process parameters and procedures which may be employed in the alkylation of aromatic compounds in accordance with the present invention may be found in U.S. Patent No. 4,744,379 to Butler et al.

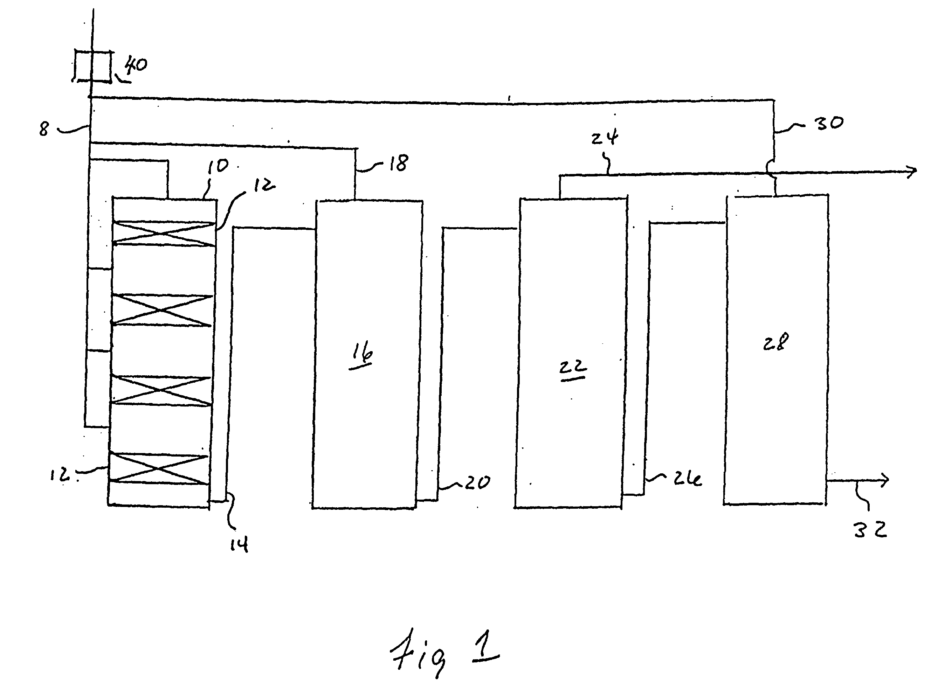

Referring now to FIGURE 1, an example of a process design suitable for performing an alkylation/transalkylation reaction according to the process of the present invention can be seen. A feed stream containing aromatic hydrocarbons such as benzene is typically passed through drying column 40. The benzene can contain as much as 700 ppm water, but current industry standards demand that the maximum water content of the aromatic feed stock comprise less than 50 ppm water. Typically, the drying column 40 separates the water and the benzene by distillation, although other methods of drying feed stock are possible.

An alkylating agent, such as ethylene, is co-injected into the reactor containing multiple beds of catalyst 12. Since the alkylation reaction is highly exothermic, the reactants may be injected at multiple locations in the reactor for process control purposes. An effluent stream 14 from the reactor 10 is directed to one or more benzene fractionating columns 16 where benzene is separated from the reactor effluent. The overhead stream 18 from the benzene fractionating column is recycled to the feed stream 8 and to the alkylation reactor 10. The bottom stream 20 from the benzene fractionating column 16 is directed to an ethylbenzene fractionating column 22 where an ethylbenzene product stream 24 is separated and recovered. The bottom stream 26 from the ethylbenzene fractionating column is directed to a third fractionating column 28 where an overhead stream 30 containing polyalkylated aromatics, is separated and recycled to the alkylation reactor for transalkylation. This recycled polyalkylated aromatic stream may contain for example, di- and tri-ethyl benzenes, xylenes, styrene, cumene and propyl benzene. Alternately, the polyalkylated aromatic stream may be directed to a separate transalkylation unit (not shown).

Higher boiling point residual materials are purged from the third fractionating column 28 in purge stream 32. The purge stream may contain such compounds as naphthalene, di- and triphenyl methane and ethane, and other heavier aromatic compounds.

Silicalite type zeolites have been found to be suitable catalysts for the alkylation/transalkylation process. Applicant has investigated alkylation/transalkylation processes using silicalite catalyst with the surprising result that when used in the above-described process, the stability of the catalyst was not deteriorated by the addition of water to the process. The stability was, to the contrary, increased. As used in this application, stability shall be understood to mean the ability of the catalyst to convert feedstock to desired products measured as a function of time during which the reactions proceed.

The silicalite catalysts employed in the present invention are formulated with a refractory oxide binder in accordance with conventional practice. Here, however, the refractory oxide binder has a somewhat larger pore size than is associated with binders commonly used to form molecular sieve catalyst particles. Specifically, the maximum pore size of the binder used in incorporating the silicalite catalyst into the catalyst particles has a maximum pore size of about 1,000-1,800 angstroms. The molecular sieve silicalite itself preferably has an average crystal size of about 0.5 µm or less, and preferably, 90 % of the crystals of the silicalite have a particle size of less than about 0.7 µm.

The silicalite crystal structure will normally exhibit a pore size of about 5 to 6 angstroms and normally no more than 7 angstroms.

A preferred refractory binder for use in the present invention is alumina having an average maximum pore size within the range of about 1,000-1,800 angstroms. While Applicants' invention is not to be limited by theory, it is postulated that by using a binder, such as alumina, of the requisite relatively large pore size, the catalyst is allowed to function without the binder imposing a diffusion-limiting factor as the silicalite is used as the catalyst in the alkylation reaction. In the present invention the catalyst particles themselves may be of any suitable size but typically will have a diameter of 0.3 cm (one-eighth inch).

In one experiment, a substantial amount of water (30,000 ppm) was added to the reactor so that the effect of the addition of the water could be rapidly and clearly ascertained. Table 1 shows the operational parameters used in the Example detailed below.

Laboratory scale alkylation test runs were performed using a silicalite catalyst that is 70%-100% monoclinic and 0%-30% orthorhombic silicalite. The silica/alumina ratio is 50-500 and the average crystal size is less than 0.50 µm. The catalyst is further characterized by a maximum pore size of the alumina binder of 1000-1800 Angstroms and a sodium content of the silicalite of less than about 50 ppm.

It is taught in the prior art that co-feeding steam to silicalite catalyst prolongs the alkylation cycle between regenerations. U.S. Patent Nos. 4,387,260 to Watson and 4,774,379 to Butler et al. disclose co-feeding steam at concentrations between 10,000 and 100,000 ppm water (1 %-10%) in an alkylation reactor. Some degradation was seen in the reported tests, however, as discussed above.

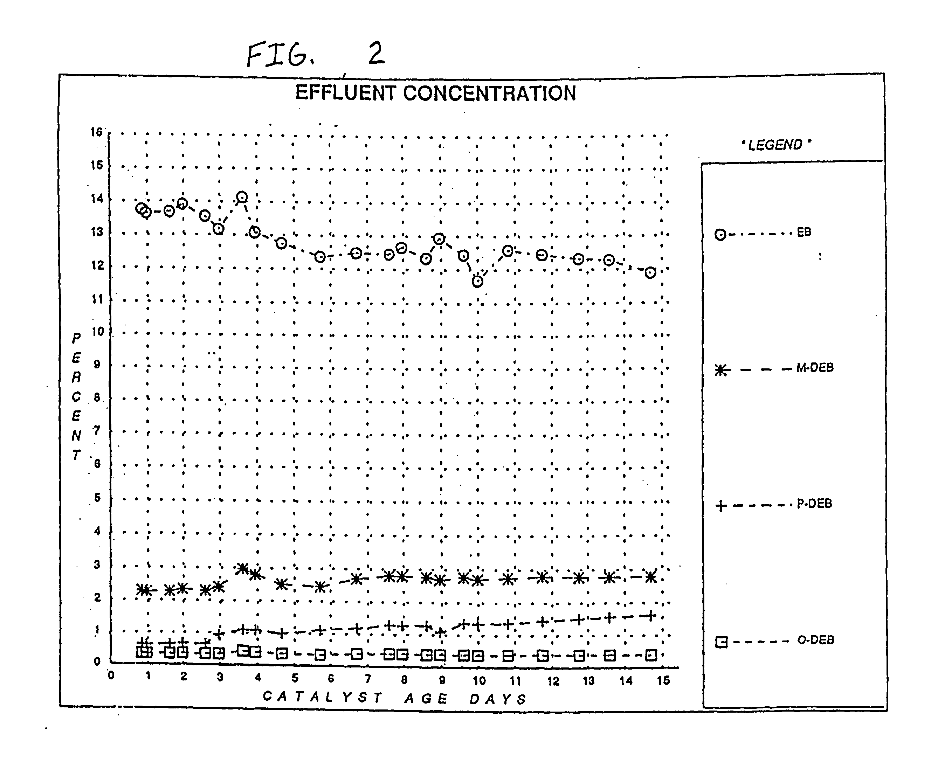

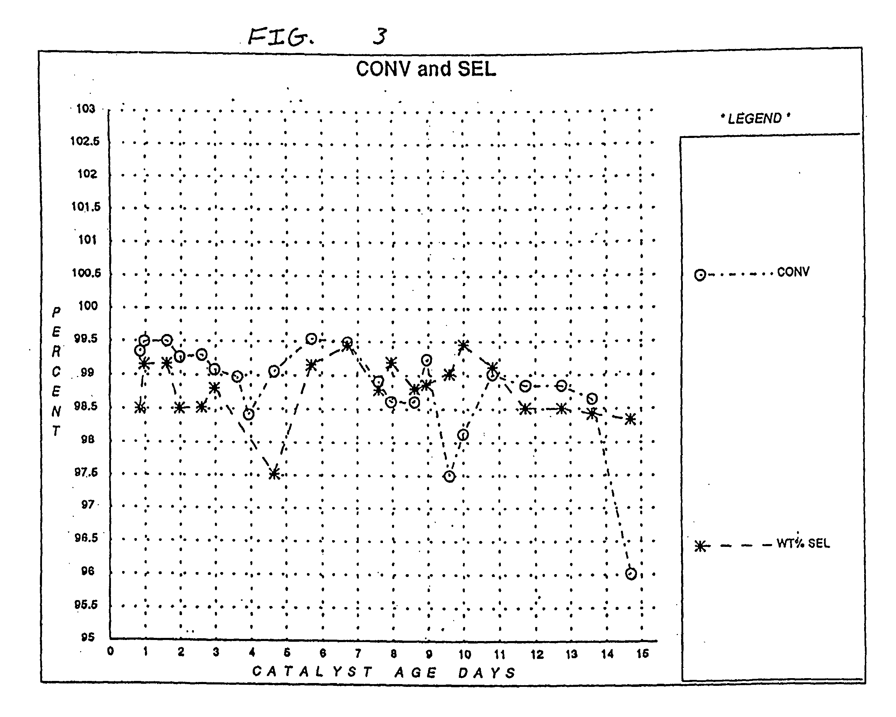

Table 2 shows the composition of the effluent stream of the laboratory reactor test run with silicalite catalyst. The composition of the alkylation reactor effluent produced by the silicalite catalyst and shown in Table 2 is within acceptable ranges. FIGURE 2 is a graphical representation of the concentrations of ethylbenzene and di-ethylbenzenes over time and FIGURE 3 represents the percent conversion and selectivity. All parameters are within expected ranges.

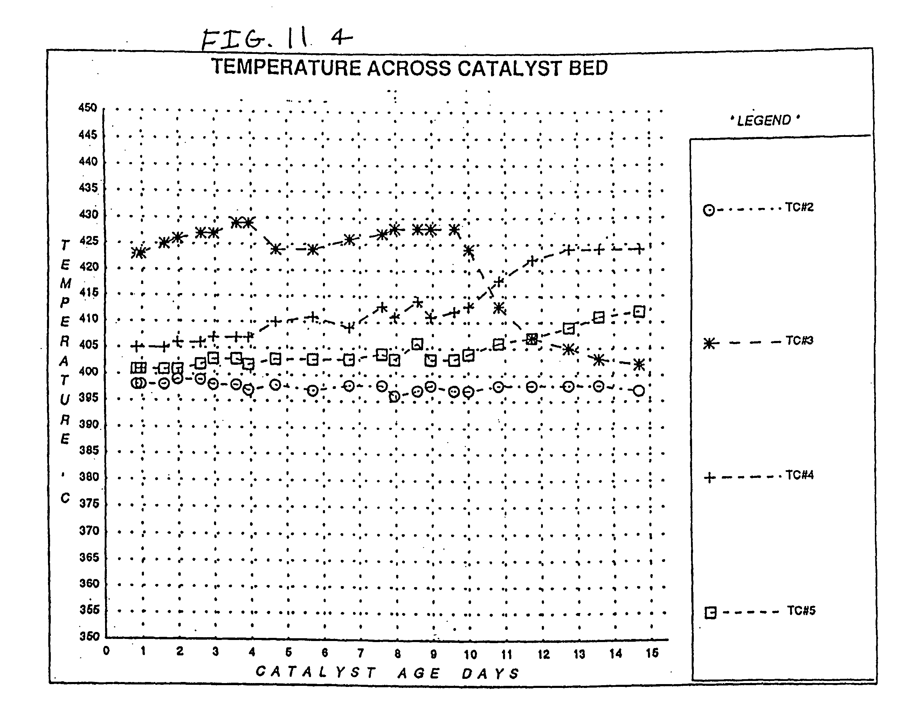

During the experimental procedure shown in Example 1 the benzene feed was interrupted on day 9 of the test, leaving only ethylene and water over the catalyst. It would be expected that the ethylene would polymerize in the catalyst immediately, causing extraordinary amounts of coking and completely shutting down the laboratory run. Remarkably, after correcting the equipment malfunction, the catalyst performed substantially as well as before the test was interrupted as evidenced by the exotherm seen in FIGURE 4.

The process of the present invention is illustrated by the follow example which is not to be construed as limiting the scope of the invention as hereinafter claimed.

### EXAMPLE

A silicalite catalyst having a particle size distribution of between 20 and 40 mesh was introduced into a laboratory scale reactor. The silicalite catalyst had a crystallinity within the range of 76-93%, 64% monoclinicity, an average crystal size of 0.41 µm, a pore volume maximum for the binder at 1763 Angstroms, and a Si/Al atomic ratio of 114. In the experimental work, the catalyst is heated under nitrogen flow to 150°C overnight at ambient pressure to dry the catalyst. The temperature is increased to 200°C and benzene with about 10% mixed polyalkylated benzenes is introduced to the reactor at a rate of 11.6 ml/min. The pressure is increased to 20,68 bar (300 psig) and the temperature raised to 400°C. After the temperature across the reactor bed has stabilized, ethylene is introduced at a rate of 1 mole for 10 moles of benzene feed. The reactor was allowed to run at normal conditions for 2 days to obtain baseline conditions. On the second day, the water pump was started for water co-feed at the rate of 0.31 ml/min or 3% by weight of benzene feed. The test was run for 14 days. The test protocol is summarized below:

**TABLE 1 -**

| TEST PROTOCOL | |
|---|---|
| PARAMETER | VALUE |
| Temperature | 400°C |
| Pressure | 20.68 bar (300 psig) |
| Feed Rate | 11.6 ml/min |
| Benzene LHSV | 70 hr⁻¹ |
| Ethylene rate | 291 st. ml/min |
| Water rate | 0.31 ml/min |

The results of the test in terms of the composition of the effluent is set forth in Table 2 below.

**TABLE 2-**

| EFFLUENT COMPOSITION | | | | |
|---|---|---|---|---|
| Test Duration (Days) | Ethylbenzene w % | Benzene wt % | M-Di-Ethylbenzene wt % | Conversion % |
| 1 | 13.74 | 79.14 | 2.29 | 99.3 |
| 2 | 13.64 | 79.31 | 2.26 | 99.5 |
| 3 | 13.68 | 79.24 | 2.28 | 99.5 |
| 4 | 13.91 | 78.82 | 2.34 | 99.3 |
| 5 | 13.56 | 79.29 | 2.31 | 99.3 |
| 6 | 13.16 | 79.28 | 2.42 | 99.1 |
| 7 | 14.13 | 76.57 | 2.97 | 99.0 |
| 8 | 13.07 | 78.10 | 2.80 | 98.4 |
| 9 | 12.76 | 79.34 | 2.51 | 99.1 |
| 10 | 12.36 | 79.87 | 2.45 | 99.5 |
| 11 | 12.49 | 78.93 | 2.71 | 99.5 |
| 12 | 12.46 | 78.66 | 2.80 | 98.9 |
| 13 | 12.66 | 78.45 | 2.80 | 99.2 |
| 14 | 12.34 | 78.84 | 2.77 | 98.8 |

## Claims

1. A process for the alkylation of aromatic compounds, the steps comprising:
(a) supplying a feedstock containing an aromatic substrate into a reaction zone, the feedstock containing from 100 to 60,000 ppm water;
(b) contacting the feedstock in the reaction zone with a silicalite catalyst formulated with a refractory oxide binder having a maximum pore size of 1,000-1,800 angstroms, said silicalite having from 70-100 percent monoclinic symmetry;
(c) supplying an alkylating agent to the reaction zone;
(d) operating the reaction zone at temperature and pressure conditions to effect alkylation of the aromatic substrate by the alkylating agent; and
(e) recovering alkylated aromatic substrate from the reaction zone.

2. The process of Claim 1 wherein the feedstock contains 100-700 ppm water.

3. The process of Claim 1 wherein the aromatic substrate comprises benzene and the alkylating agent comprises ethylene.

4. The process of Claim 1 wherein the silicalite catalyst has an average crystal size of less than about 0.50 microns and a Si/AI atomic ratio of 50-500.

5. The process of Claim 4 wherein the silicalite catalyst has a sodium content of less than about 50 ppm.

6. The process of Claim 4 wherein 90% of the crystals of the silicalite catalyst have a particle size less than about 0.70 µm.

7. The process of Claim 1 wherein the temperature and pressure conditions include temperatures of from 250°C to 500°C and pressures of from 13.79 bars (200 psi) to 34.47 bars (500 psi).

8. The process of Claim 1 wherein the feedstock also comprises polyalkylated aromatic compounds.

9. The process of Claim 8 wherein the polyalkylated aromatic compounds comprise substantially 10% of the feedstock.

## Patentansprüche

1. Verfahren für die Alkylierung von aromatischen Verbindungen, die Stufen umfassend:
(a) Liefern eines Einsatzmaterials, enthaltend ein aromatisches Substrat, in eine Reaktionszone, wobei das Einsatzmaterial 100 bis 60 000 ppm Wasser enthält,
(b) in Kontakt bringen des Einsatzmaterials in der Reaktionszone mit einem Silicalitkatalysator, formuliert mit einem feuerfesten Oxidbindemittel mit einer Maximumporengröße von 1 000 bis 1 800 Angstrom, wobei der Silicalit 70-100 Prozent monokline Symmetrie hat,
(c) Liefern eines Alkylierungsmittels zu der Reaktionszone,
(d) Betreiben der Reaktionszone bei Temperatur- und Druckbedingungen unter Bewirken von Alkylierung des aromatischen Substrats durch das Alkylierungsmittel, und
(e) Gewinnen von alkyliertem aromatischem Substrat aus der Reaktionszone.

2. Verfahren nach Anspruch 1, wobei das Einsatzmaterial 100 - 700 ppm Wasser enthält.

3. Verfahren nach Anspruch 1, wobei das aromatische Substrat Benzol umfaßt, und das Alkylierungsmittel umfaßt Ethylen.

4. Verfahren nach Anspruch 1, wobei der Silicalitkatalysator eine Durchschnittskristallgröße von weniger als 0,5 Mikrometer und ein Si/Al Atomverhältnis von 50-500 hat.

5. Verfahren nach Anspruch 4, wobei der Silicalitkatalysator einen Natriumgehalt von weniger als etwa 50 ppm hat.

6. Verfahren nach Anspruch 4, wobei 90% der Kristalle des Silicalitkatalysators eine Teilchengröße von weniger als etwa 0,70 µm haben.

7. Verfahren nach Anspruch 1, wobei die Temperatur- und Druckbedingungen Temperaturen von 250°C bis 500°C und Drucke von 13,79 Bar (200 psi) bis 34,47 Bar (500 psi) einschließen.

8. Verfahren nach Anspruch 1, wobei das Einsatzmaterial auch polyalkylierte aromatische Verbindungen umfaßt.

9. Verfahren nach Anspruch 8, wobei die polyalkylierten aromatischen Verbindungen im wesentlichen 10% des Einsatzmaterials umfassen.

## Revendications

1. Procédé pour l'alkylation de composés aromatiques, comprenant les étapes consistant à :
(a) acheminer une matière d'alimentation contenant un substrat aromatique dans une zone de réaction, la matière d'alimentation contenant de l'eau à concurrence de 100 à 60.000 ppm ;
(b) mettre la matière d'alimentation dans la zone de réaction en contact avec un catalyseur à base de silicalite qui a été formulé avec un liant d'oxyde réfractaire possédant une dimension de pore maximale de 1000 à 1800 Angstrôm, ladite silicalite possédant une symétrie monoclinique à raison de 70 à 100 % ;
(c) acheminer un agent d'alkylation à la zone de réaction ;
(d) mettre en service la zone de réaction dans des conditions de température et de pression nécessaires pour l'alkylation du substrat aromatique par l'agent d'alkylation ; et
(e) récupérer à partir de la zone de réaction le substrat aromatique alkylé.

2. Procédé selon la revendication 1, dans lequel la matière d'alimentation contient de l'eau à concurrence de 100 à 700 ppm.

3. Procédé selon la revendication 1, dans lequel le substrat aromatique comprend du benzène et l'agent d'alkylation comprend de l'éthylène.

4. Procédé selon la revendication 1, dans lequel le catalyseur à base de silicalite possède une dimension de cristal moyenne inférieure à environ 50 microns et un rapport atomique Si/Al de 50 à 500.

5. Procédé selon la revendication 4, dans lequel le catalyseur à base de silicalite possède une teneur en sodium inférieure à environ 50 ppm.

6. Procédé selon la revendication 4, dans lequel 90 % des cristaux du catalyseur à base de silicalite possèdent une granulométrie inférieure à environ 0,70 µm.

7. Procédé selon la revendication 1, dans lequel les conditions de température et de pression englobent des températures de 250 °C à 500 °C et des pressions de 13,79 bars (200 psi) à 34,47 bars (500 psi).

8. Procédé selon la revendication 1, dans lequel la matière d'alimentation comprend également des composés aromatiques polyalkylés.

9. Procédé selon la revendication 8, dans lequel les composés aromatiques polyalkylés comprennent essentiellement 10 % de la matière d'alimentation.
